(19) Europäisches Patentamt
European Patent Office
Office européen des brevets

(11) EP 2 883 869 A1

(12) EUROPEAN PATENT APPLICATION
published in accordance with Art. 153(4) EPC

(43) Date of publication:
**17.06.2015 Bulletin 2015/25**

(21) Application number: **13828290.0**

(22) Date of filing: **05.08.2013**

(51) Int Cl.:
***C07D 233/64*** $^{(2006.01)}$ ***C07D 235/18*** $^{(2006.01)}$

(86) International application number:
**PCT/JP2013/071147**

(87) International publication number:
**WO 2014/024844 (13.02.2014 Gazette 2014/07)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **10.08.2012 JP 2012178482**

(71) Applicant: **Daicel Corporation**
**Osaka-shi, Osaka 530-0001 (JP)**

(72) Inventor: **FUJITA, Kouhei**
**Himeji-shi**
**Hyogo 671-1283 (JP)**

(74) Representative: **Grünecker Patent- und Rechtsanwälte**
**PartG mbB**
**Leopoldstraße 4**
**80802 München (DE)**

# (54) CRYSTALS CONTAINING UNSATURATED CARBOXYLIC ACID AMIDE COMPOUND AND METHOD FOR PRODUCING SAME

(57) Provided is a crystal of an unsaturated carboxylic acid amide. The crystal is useful as or for fine chemicals such as pharmaceuticals, agricultural chemicals, polymeric materials, functional materials, and intermediates of them, has a high purity, still is not bulky, and can be handled excellently.

The crystal includes an unsaturated carboxylic acid amide represented by Formula (1) in an amount of 95 percent by area or more and has a bulk density of 0.2 to 0.7 g/mL. In the formula, $R^1$ to $R^5$ are independently selected from hydrogen, alkyl, hydroxyl, alkoxy, and nitro. At least two of $R^1$ to $R^5$ may be linked to form a ring together with carbon atoms constituting the specified aromatic ring. $R^6$, $R^7$, and $R^8$ are independently selected from hydrogen, alkyl, and aryl. $R^7$ and $R^8$ may be linked to form a ring together with carbon atoms constituting the specified imidazole ring.
[Chem. 1]

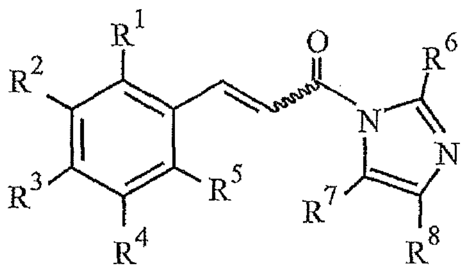

(1)

EP 2 883 869 A1

## Description

### Technical Field

The present invention relates to crystals including unsaturated carboxylic acid amide compounds, and methods for producing such crystals. The unsaturated carboxylic acid amide compounds are useful as fine chemicals such as pharmaceuticals, agricultural chemicals, polymeric materials, functional materials, and intermediates of them.

### Background Art

Unsaturated carboxylic acid amide compounds have been produced typically by methods in which an unsaturated carboxylic acid is dehydratively condensed with an amine. Patent Literature (PTL) 1 describes a method of dehydratively condensing 2-hydroxycinnamic acid with an amine using a carbodiimide as a dehydrating-condensing agent to give 2-hydroxycinnamamide. Non Patent Literature (NPL) 1 describes a method of dehydratively condensing 4-methoxycinnamic acid with an amine using N,N-carbonyldiimidazole as a dehydrating-condensing agent to give 4-methoxycinnamic acid amide. Unfortunately, the dehydrating-condensing agents used in these methods are expensive and may cause a severe allergic reaction. The methods are therefore not considered as industrially advantageous methods. Independently, unsaturated carboxylic acid amides have been produced by a method of hydrating unsaturated carboxylic acid nitriles. Unfortunately, however, the method suffers typically from inferior reaction selectivity in some kinds of the unsaturated carboxylic acid moiety and is not always considered as a popular or general method.

Under such circumstances, unsaturated carboxylic acid amides are often synthetically produced by a method of allowing an unsaturated carboxylic acid chloride to react with an amine. The unsaturated carboxylic acid chloride is most generally produced by a method of allowing an unsaturated carboxylic acid to react with thionyl chloride. The method is advantageous typically in that thionyl chloride has a relatively low boiling point and, if remained in excess, can be easily removed. In a method described in NPL 2, an unsaturated carboxylic acid is mixed with 8.6 times the mole of thionyl chloride with cooling and, after the initiation of reaction, the mixture is heated to reflux to give an unsaturated carboxylic acid chloride.

Specifically, an unsaturated carboxylic acid amide is generally produced by a method in which an unsaturated carboxylic acid is allowed to react with thionyl chloride to synthetically give an unsaturated carboxylic acid chloride, and the prepared unsaturated carboxylic acid chloride is allowed to react with an amine to synthetically give the target unsaturated carboxylic acid amide.

Independently, highly-functional materials (high-performance materials) include photosensitive high-performance materials (resist materials) for use in the production of electrical and electronic components. Among them, those particularly requiring high reliability include semiconductor encapsulating materials; packaging materials for semiconductors (semiconductor devices) and MEMS (micro electro mechanical systems); and photosensitive materials for semiconductor devices, liquid crystal displays, and MEMS. These materials should include ingredients with higher and higher purity. Of impurities or by-products, chlorine-containing compounds are known to significantly affect the performance of electrical and electronic components. PTL 2 discloses that a halogen atom contained in by-products becomes a halogen anion upon exposure (light irradiation), thus reduces the effect of an acid generator, and lowers the sensitivity. PTL 3 discloses as follows. Assume that a compound contains, as impurities, large amounts of organic chlorine compounds such as hydrolyzable chlorine that liberates a chlorine ion upon moisture absorption. This compound, when used in the production of an electrical/electronic component, causes the electrical/electronic component to be susceptible to corrosion and/or a break of interconnections and deterioration in insulation quality and to have inferior reliability.

For these reasons, it is important to perform purification treatment so as to give a crystal including an unsaturated carboxylic acid amide with a high purity. Disadvantageously, however, conventional purification processes give crystals of unsaturated carboxylic acid amide compounds that are fluffy and bulky with a low bulk density, although they have a high purity. Thus, the crystals are not handled satisfactorily. Typically, the crystals should be dried in a large dryer or be dried in small divided portions in multiple times. In addition, the crystals have poor properties in transportation and filling (charging).

### Citation List

#### Patent Literature

PTL 1: PCT International Publication Number WO2009/123122

PTL 2: Japanese Unexamined Patent Application Publication (JP-A) No. 2002-187868

PTL 3: JP-A No. 2009-263543

Non Patent Literature

NPL 1: Journal of Organic Chemistry, volume 69, p. 4216 (2004)
NPL 2: Journal of American Chemical Society, volume 72, p. 3885 (1950)
NPL 3: Synthesis, p. 598 (1989)

Summary of Invention

Technical Problem

Accordingly, it is an object of the present invention to provide a crystal of an unsaturated carboxylic acid amide, where the crystal is useful as fine chemicals, has a high purity, is not bulky, and can be handled excellently. The fine chemicals are exemplified by pharmaceuticals, agricultural chemicals, polymeric materials, functional materials, and intermediates of them.

It is another object of the present invention to provide a method for producing the crystal of the unsaturated carboxylic acid amide, where the crystal has a high purity, is not bulky, and can be handled excellently. Solution to Problem

After intensive investigations to achieve the objects, the present inventor has found as follows. Assume that an unsaturated carboxylic acid amide is produced by a method that includes chlorinating an unsaturated carboxylic acid with thionyl chloride to give an unsaturated carboxylic acid chloride, and allowing the unsaturated carboxylic acid chloride to react with an imidazole compound. Further assume that the production method further includes control of the amount of thionyl chloride within a specific range, or further includes a reaction step of purifying a reaction product using an adsorbent after the reaction. This production method can yield an unsaturated carboxylic acid amide having a very low chlorine-containing compound content. Still further assume that the production method further includes, after the reaction, washing with water, azeotropic dehydration to a water content of 0.3 percent by weight or less, and crystallization in the specified sequence. This production method can give a crystal of an unsaturated carboxylic acid amide, where the crystal has a high purity and is not bulky. The present invention has been made based on these findings.

Specifically, the present invention provides, in one aspect, a crystal including an unsaturated carboxylic acid amide in an amount of 95 percent by area or more and having a bulk density of from 0.2 to 0.7 g/mL. The unsaturated carboxylic acid amide is represented by Formula (1):
[Chem. 1]

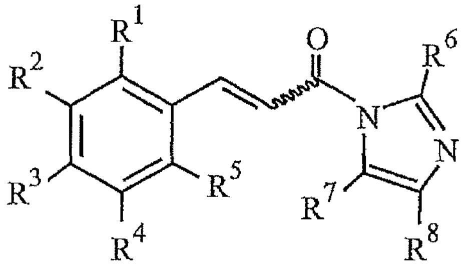

(1)

where $R^1$ to $R^5$ are, identically or differently, selected from hydrogen, alkyl, hydroxyl, alkoxy, and nitro. At least two of $R^1$ to $R^5$ may be linked to form a ring together with carbon atoms constituting the specified aromatic ring. $R^6$, $R^7$, and $R^8$ are, identically or differently, selected from hydrogen, alkyl, and aryl. $R^7$ and $R^8$ may be linked to form a ring together with carbon atoms constituting the specified imidazole ring. The amount of the unsaturated carboxylic acid amide is measured by gas chromatography under analysis conditions as follows. Specifically, the gas chromatographic analysis is performed using a DB-1701 column of 0.25 mm in diameter by 30 m in length, at a film thickness of 0.25 $\mu$m, a split ratio of 1:50, a flow rate of 1 mL/min (He), an injection volume of 1 $\mu$L, an inlet temperature of 250°C, and a detector temperature of 280°C, with a temperature program of holding at 50°C for 4 minutes, subsequently heating at a rate of 15°C/min up to 280°C, and holding at 280°C for 10 minutes. The analysis is performed for an analysis time of 30 min.

Preferably, the crystal, in X-ray diffraction, exhibits a peak at 28 in at least one range selected from the group consisting of 6.0 to 8.0, 13.5 to 15.5, and 16.5 to 18.0 and exhibits approximately no peak at 2$\theta$ in ranges of 9.0 to 10.5, 12.5 to 13.5, and 29.0 to 30.0.

The unsaturated carboxylic acid amide preferably includes at least one compound selected from compounds

represented by Formulae (1-1) to (1-6):
[Chem. 2]

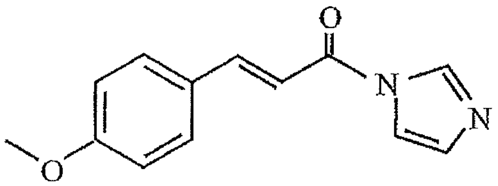

(1-1)

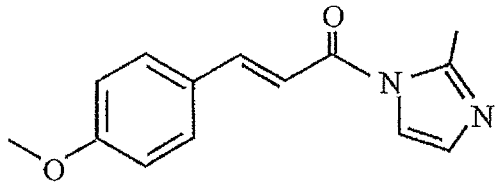

(1-2)

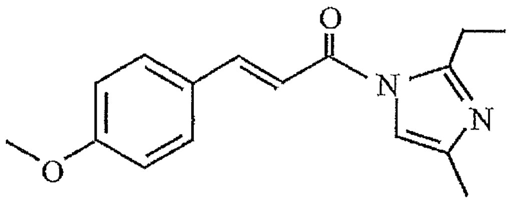

(1-3)

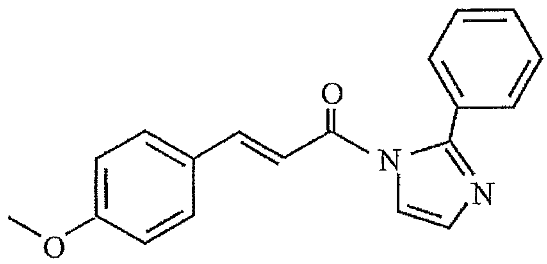

(1-4)

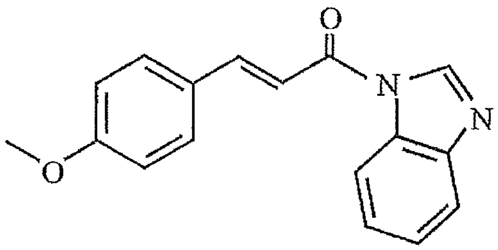

(1-5)

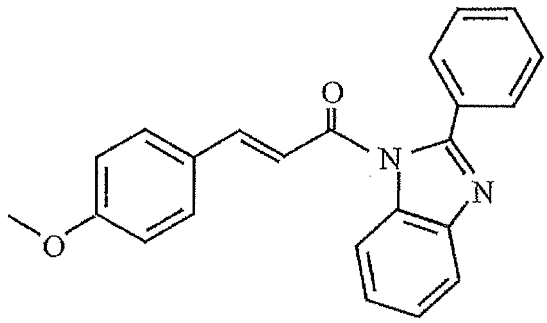

(1-6)

In addition and advantageously, the present invention provides a method for producing the crystal. The method includes a reaction step 1 (step a)), a reaction step 2 (step b)), and a purification step (step c)). In the reaction step 1, an unsaturated carboxylic acid represented by Formula (2) is chlorinated with thionyl chloride to give a compound represented by Formula (3). In the reaction step 2, the prepared compound represented by Formula (3) is allowed to react with an imidazole compound represented by Formula (4) to give an unsaturated carboxylic acid amide represented by Formula (1) as a reaction product. In the purification step, the reaction product is purified to give the target crystal. The method includes at least one of using thionyl chloride in an amount of 0.5 to 3.0 times the mole of the unsaturated carboxylic acid represented by Formula (2) in the reaction step 1; and separating/removing a chlorine-containing compound using an adsorbent in the purification step. The purification step includes adding water to the reaction system to wash the reaction product. The reaction product after washing is azeotropically dehydrated to a water content of 0.3 percent by weight or less, and is then subjected to crystallization. Formulae (2), (3), (4), and (1) are expressed as follows:
[Chem. 3]

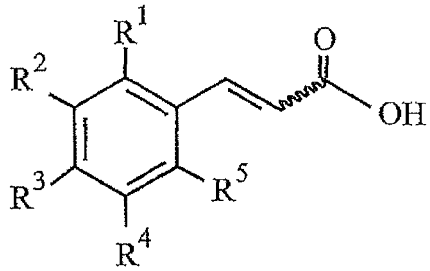

(2)

where $R^1$ to $R^5$ are, identically or differently, selected from hydrogen, alkyl, hydroxyl, alkoxy, and nitro, where at least two of $R^1$ to $R^5$ may be linked to form a ring together with carbon atoms constituting the specified aromatic ring,
[Chem. 4]

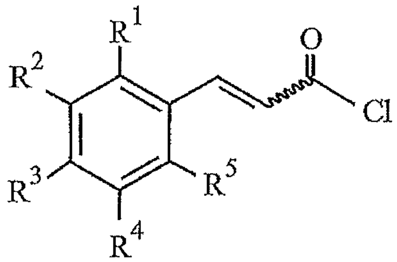

(3)

where $R^1$ to $R^5$ are as defined above,
[Chem. 5]

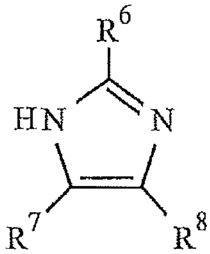

(4)

where $R^6$, $R^7$, and $R^8$ are, identically or differently, selected from hydrogen, alkyl, and aryl, where $R^7$ and $R^8$ may be linked to form a ring together with carbon atoms constituting the specified imidazole ring,
[Chem. 6]

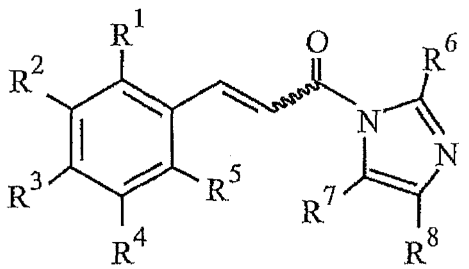

(1)

where $R^1$ to $R^8$ are as defined above.

Advantageous Effects of Invention

The crystal according to the present invention includes the unsaturated carboxylic acid amide represented by Formula (1) in an amount of 95 percent by area or more and has a bulk density of from 0.2 to 0.7 g/mL. The crystal has a high purity, is not bulky, and can be handled excellently (e.g., can be processed and transported excellently). The

crystal is therefore useful particularly as raw materials for industrial products and is advantageously usable as or for pharmaceuticals, agricultural chemicals, polymeric materials, high-performance materials, and intermediates of them. The high-performance materials are exemplified by photosensitive high-performance materials (resist materials) for use in the production of electrical and electronic components. In particular, the crystal is advantageously usable as or for materials requiring high reliability, such as semiconductor encapsulating materials, packaging materials typically for semiconductor devices and MEMS
(micro electro mechanical systems), and photosensitive materials for semiconductor devices, liquid crystal displays, and MEMS.

Brief Description of Drawings

[Fig. 1] Fig. 1 is an X-ray diffraction (XRD) chart of crystals prepared in Example 1.
[Fig. 2] Fig. 2 is an X-ray diffraction (XRD) chart of crystals prepared in Comparative Example 1.

Description of Embodiments

Crystal

The crystal according to the present invention includes an unsaturated carboxylic acid amide represented by Formula (1) in an amount of 95 percent by area or more and has a bulk density of from 0.2 to 0.7 g/mL. Formula (1) is expressed as follows:
[Chem. 7]

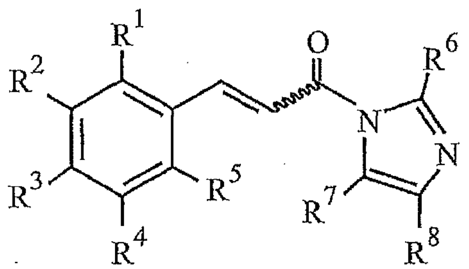
(1)

where $R^1$ to $R^5$ are, identically or differently, selected from hydrogen, alkyl, hydroxyl, alkoxy, and nitro. At least two of $R^1$ to $R^5$ may be linked to form a ring together with carbon atoms constituting the specified aromatic ring. $R^6$, $R^7$, and $R^8$ are, identically or differently, selected from hydrogen, alkyl, and aryl. $R^7$ and $R^8$ may be linked to form a ring together with carbon atoms constituting the specified imidazole ring.

The crystal according to the present invention may be produced by a method as follows. The method includes a reaction step 1, a reaction step 2, and a purification step. In the reaction step 1, an unsaturated carboxylic acid represented by Formula (2) is chlorinated with thionyl chloride to give a compound represented by Formula (3). In the reaction step 2, the prepared compound represented by Formula (3) is allowed to react with an imidazole compound represented by Formula (4) to give an unsaturated carboxylic acid amide represented by Formula (1). Formulae (2), (3), (4), and (1) are expressed as follows:
[Chem. 8]

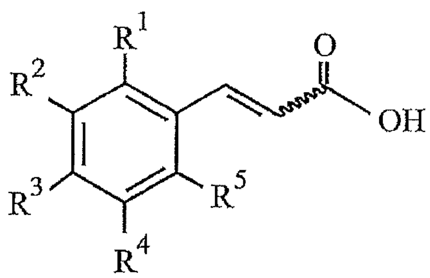
(2)

where $R^1$ to $R^5$ are, identically or differently, selected from hydrogen, alkyl, hydroxyl, alkoxy, and nitro, where at least

two of $R^1$ to $R^5$ may be linked to form a ring together with carbon atoms constituting the specified aromatic ring,
[Chem. 9]

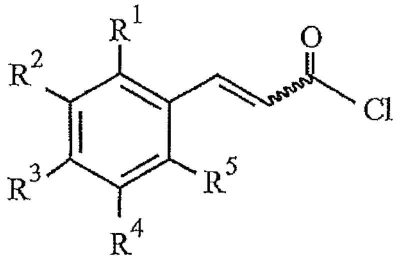

(3)

where $R^1$ to $R^5$ are as defined above,
[Chem. 10]

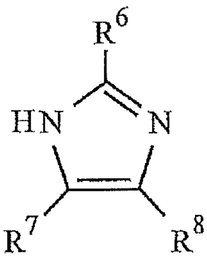

(4)

where $R^6$, $R^7$, and $R^8$ are, identically or differently, selected from hydrogen, alkyl, and aryl, where $R^7$ and $R^8$ may be linked to form a ring together with carbon atoms constituting the specified imidazole ring,
[Chem. 11]

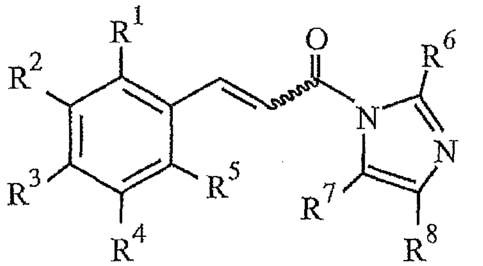

(1)

where $R^1$ to $R^8$ are as defined above.
The method is performed under both conditions 1 and 2 as follows.

1. In the reaction step 1, thionyl chloride is used in an amount 0.5 to 3.0 times the mole of the unsaturated carboxylic acid represented by Formula (2), and/or, in the purification step, a chlorine-containing compound is separated and removed using an adsorbent.
2. The purification step includes washing the reaction system with water. The resulting mixture is azeotropically dehydrated to a water content of 0.3 percent by weight or less, and then subjected to crystallization.

As $R^1$ to $R^5$, the alkyl is exemplified by alkyl containing 1 to about 4 carbon atoms, such as methyl, ethyl, propyl, isopropyl, and butyl. The alkoxy is exemplified by alkoxy containing 1 to about 6 carbon atoms, such as methoxy, ethoxy, isopropoxy, butoxy, isobutoxy, sec-butoxy, tert-butoxy, and pentoxy. In an embodiment, at least two of $R^1$ to $R^5$ are linked to form a ring together with carbon atoms constituting the specified aromatic ring. The formed ring is exemplified by aromatic rings containing about 6 to about 20 carbon atoms, such as benzene, naphthalene, and anthracene rings, of which those containing 6 to 14 carbon atoms are preferred; and hydrocarbon rings (e.g., cycloalkane, cycloalkene, and bridged carbon rings) containing 3 to about 20 members, such as cyclobutane, cyclopentane, cyclohexane, cyclohexene, cyclooctane, cyclododecane, adamantane, norbornane, and norbornene rings, of which those containing 3 to 15 members are preferred, and those containing 5 to 12 members are particularly preferred. In a preferred embodiment

of the present invention, $R^1$, $R^2$, $R^4$, and $R^5$ are hydrogen, and $R^3$ is methoxy. This can give a compound having excellent absorption sensitivity from easily available raw materials.

As $R^6$, $R^7$, and $R^8$, the alkyl is exemplified by alkyl containing 1 to about 20 carbon atoms, such as methyl, ethyl, propyl, isopropyl, butyl, isobutyl, s-butyl, t-butyl, pentyl, hexyl, decyl, and dodecyl, of which those containing 1 to 10 carbon atoms are preferred. The aryl is exemplified by aryl containing about 6 to about 20 carbon atoms, such as phenyl and naphthyl, of which those containing 6 to 14 carbon atoms are preferred.

In an embodiment, $R^7$ and $R^8$ are linked to form a ring together with carbon atoms constituting the specified imidazole ring. The formed ring is exemplified by aromatic rings such as benzene ring. The ring may have one or more substituents. The substituents are exemplified by alkyl containing 1 to about 3 carbon atoms, such as methyl, ethyl, and propyl; and aryl containing about 6 to about 20 carbon atoms, such as phenyl and naphthyl, of which those containing 6 to 14 carbon atoms are preferred.

Specifically, the imidazole compound represented by Formula (4) is exemplified by imidazole, 2-methylimidazole, 2-ethyl-4-methylimidazole, 2-phenylimidazole, benzimidazole, and 2-phenylbenzimidazole.

Also specifically, the unsaturated carboxylic acid amide represented by Formula (1) is exemplified by compounds represented by Formulae (1-1) to (1-6) below. In particular, the crystal according to the present invention preferably includes the compound represented by Formula (1-1).

[Chem. 12]

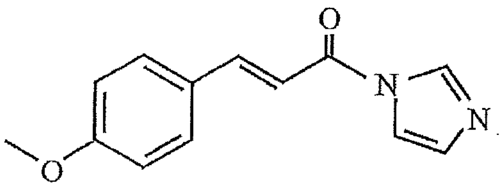

(1-1)

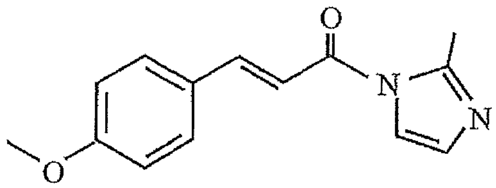

(1-2)

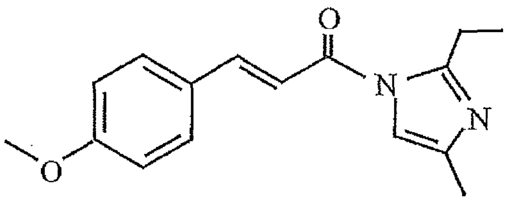

(1-3)

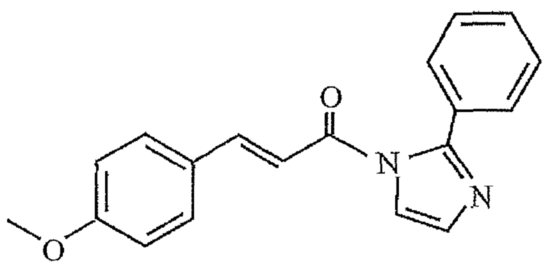

(1-4)

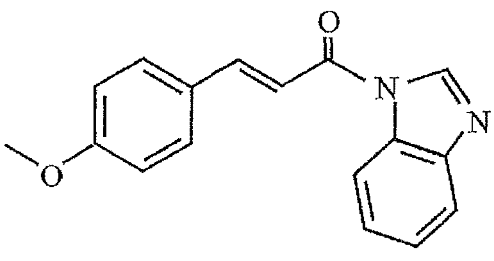

(1-5)

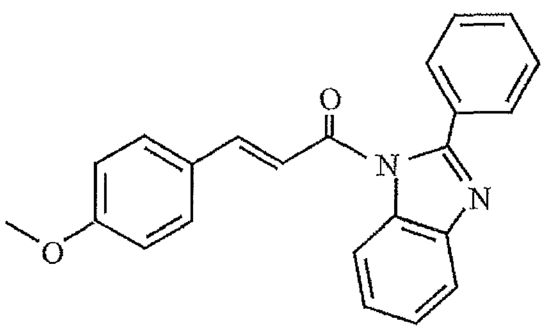

(1-6)

Reaction Step 1

The reaction step 1 is the step of chlorinating the unsaturated carboxylic acid represented by Formula (2) with thionyl chloride to give the compound represented by Formula (3).

The thionyl chloride is used in an amount of from about 0.5 to about 3.0 times, preferably from 0.8 to 2.5 times, more preferably from 0.9 to 1.8 times, particularly preferably from 1.0 to 1.5 times, and most preferably from 1.0 to 1.3 times, the mole of the unsaturated carboxylic acid represented by Formula (2). The thionyl chloride, if used in an amount greater than the range, may readily cause chlorine-containing compounds to be by-produced in larger amounts. This may impede the use of the target product as a photosensitive high-performance material. In contrast, the thionyl chloride, if used in an amount less than the range, may readily cause the target product to be obtained in a lower yield. As used herein the term "chlorine-containing compound(s)" refers to all compounds that contain chlorine and are by-produced in the method for producing an unsaturated carboxylic acid amide according to the present invention. Major chlorine-containing compounds are exemplified by chlorinated derivatives (chlorides) of the unsaturated carboxylic acid amide represented by Formula (1), and analogues of them.

The chlorination reaction may be performed in the presence of, or in the absence of, a solvent. The solvent is exemplified by saturated or unsaturated hydrocarbon solvents such as pentane, hexane, heptane, octane, and petroleum ether; aromatic hydrocarbon solvents such as benzene, toluene, and xylenes; halogenated hydrocarbon solvents such as methylene chloride, chloroform, 1,2-dichloroethane, chlorobenzene, and bromobenzene; ether solvents such as diethyl ether, diisopropyl ether, dibutyl ether, tetrahydrofuran, dioxane, 1,2-dimethoxyethane, and cyclopentyl methyl ether; nitrile solvents such as acetonitrile and benzonitrile; sulfoxide solvents such as dimethyl sulfoxide; sulfolane solvents such as sulfolane; amide solvents such as dimethylformamide; and high-boiling solvents such as silicone oils. Each of them may be used alone or in combination.

Among them, aromatic hydrocarbon solvents and halogenated hydrocarbon solvents are particularly preferably usable. Such an aromatic hydrocarbon solvent and/or a halogenated hydrocarbon solvent may be used in an amount of typically 10 percent by weight or more, preferably 30 percent by weight or more, particularly preferably 50 percent by weight or more, and most preferably 80 percent by weight or more, of the total amount (100 percent by weight) of all solvents used in the chlorination reaction. The "amount" herein refers to a "total amount" when two or more solvents selected from aromatic hydrocarbon solvents and halogenated hydrocarbon solvents are used in combination.

The solvent(s) may be used in an amount of typically from about 1 to about 100000 parts by weight, and preferably from 1 to 10000 parts by weight, per 1 part by weight of the unsaturated carboxylic acid represented by Formula (2), although the amount is not critical, within such a range as to allow dissolution or dispersion of reactants and as not to impair properties such as economic efficiency.

The chlorination reaction may be performed typically by adding thionyl chloride dropwise to a system including the unsaturated carboxylic acid represented by Formula (2). The reaction is performed for a reaction time of typically from about 0.5 to about 48 hours, preferably from 1 to 36 hours, and particularly preferably from 2 to 24 hours. The thionyl chloride dropwise addition may be performed at a temperature of typically from 40°C up to the boiling point(s) of substances present in the reaction system, preferably from 55°C to 120°C, and particularly preferably from 60°C to 75°C. The reaction after the completion of thionyl chloride dropwise addition may be performed at a temperature of typically from 55°C up to the boiling point(s) of substances present in the reaction system, preferably from 55°C to 120°C, and particularly preferably from 60°C to 75°C. The thionyl chloride dropwise addition and the reaction after the completion of dropwise addition may be performed at identical or different temperatures. The thionyl chloride dropwise addition and the reaction after the completion of dropwise addition, if each performed at a temperature lower than the range, may readily cause the compound represented by Formula (3) to be prepared in a lower yield. In contrast, the thionyl chloride dropwise addition and the reaction after the completion of dropwise addition, if each performed at a temperature higher than the range, may cause chlorine-containing compounds to be by-produced in larger amounts. This may impede the use of the resulting product as a photosensitive high-performance material.

The chlorination reaction may be performed under any of a pressure (under a load), normal atmospheric pressure, and reduced pressure (typically from about 0.0001 to about 0.1 MPa, and preferably from 0.001 to 0.1 MPa). The chlorination reaction may be often performed under normal atmospheric pressure or under reduced pressure.

The method according to the present invention preferably includes an operation of removing excessive thionyl chloride during or after the completion of the reaction step 1. This is preferred for a lower content of chlorine-containing compounds in the crystal. The removal of the excessive thionyl chloride may be performed by separation using a common process such as degassing, extraction, distillation, rectification, molecular distillation, and/or adsorption. Such process or operation may be performed continuously or discontinuously (batchwise). The operation may be performed under either of reduced pressure and normal atmospheric pressure.

In a preferred embodiment in the reaction step 1, the reaction may be performed while continuously separating a by-produced acidic gas from the reaction system, where the acidic gas is exemplified by hydrogen chloride and sulfur dioxide gases. This is preferred for a lower content of chlorine-containing compounds in the crystal. The separation of the by-produced acidic gas may be performed by separation using a common procedure such as degassing, extraction, distillation, rectification, molecular distillation, and/or adsorption. Such operation or procedure may be performed continuously or discontinuously (batchwise). The operation may be performed under either of reduced pressure and normal atmospheric pressure.

Reaction Step 2

The reaction step 2 is the step of allowing the compound represented by Formula (3) prepared in the reaction step 1 to react with the imidazole compound represented by Formula (4) to give the unsaturated carboxylic acid amide represented by Formula (1).

The imidazole compound represented by Formula (4) may be used in an amount of typically from about 0.5 to about 20.0 times, preferably from 0.8 to 8.0 times, and particularly preferably from 1.0 to 3.0 times, the mole of the compound represented by Formula (3). The imidazole compound represented by Formula (4), if used in an amount greater than the range, may readily adversely affect the reaction operability and economic efficiency. In contrast, the imidazole compound represented by Formula (4), if used in an amount less than the range, may readily cause the unsaturated carboxylic acid amide represented by Formula (1) to be prepared in a lower yield.

The reaction may be performed in the presence of, or in the absence of, a solvent. The solvent is exemplified as with the solvents usable in the reaction step 1. The solvent may be used in an amount of typically from about 1 to about 100000 parts by weight, and preferably from 1 to 10000 parts by weight, per 1 part by weight of the compound represented by Formula (3), although the amount is not critical within such a range as to allow the dissolution or dispersion of reactants and as not to adversely affect properties such as economic efficiency. The solvent used in the reaction step 1 may be used commonly as the solvent for use in the reaction step 2. In this case, the solvent used in the reaction step 1 may be used in the reaction step 2 as intact or after adjusting the concentration by concentrating or diluting after the completion of the reaction step 1.

The reaction in the reaction step 2 may be performed at a temperature of typically from -50°C to 150°C, preferably from -10°C to 80°C, and particularly preferably from 10°C to 50°C. The reaction may be performed under any of a pressure (under a load), normal atmospheric pressure, and reduced pressure (typically from about 0.0001 to about 0.1 MPa, and preferably from 0.001 to 0.1 MPa). The reaction is often performed under normal atmospheric pressure or under reduced pressure. The reaction may be performed in any system selected from batch, semi-batch, and continuous systems.

In the reaction step 2, an acidic gas (hydrogen chloride) and/or an amine hydrochloride gas is by-produced with reaction progress. In a preferred embodiment of the present invention, these substances are removed so as to accelerate the reaction progress and to inhibit the formation of by-products such as chlorine-containing compounds. A way to remove these substances is exemplified by a method of adding a base so as to trap or scavenge the substances, where the base is inert to the reaction or does not affect the obtaining of the target substance; and a method of performing the reaction while separating them from the reaction system continuously or discontinuously (batchwise) by a separation process such as degassing, extraction, distillation, rectification, molecular distillation, and/or adsorption. The separation operation may be performed under either of reduced pressure and normal atmospheric pressure.

The base that is inert to the reaction or does not affect the obtaining of the target substance is exemplified by inorganic bases such as sodium hydrogencarbonate, sodium carbonate, potassium carbonate, potassium hydrogencarbonate, sodium hydroxide, and potassium hydroxide; aromatic amines such as pyridine; primary amines such as methylamine, ethylamine, propylamine, isopropylamine, allylamine, butylamine, pentylamine, hexylamine, octylamine, 2-ethylhexylamine, benzylamine, cyclopentylamine, cyclohexylamine, aniline, toluidine, xylidine, naphthylamine, and 2-aminothiazole; secondary amines such as dimethylamine, diethylamine, dipropylamine, diisopropylamine, diallylamine, dibutylamine, dipentylamine, dioctylamine, di(2-ethylhexyl)amine, ethylenimine, pyrrolidine, piperidine, piperazine, morpholine, N-methylaniline, diphenylamine, phenothiazine, and pyrazole; and tertiary amines such as triethylamine and tributylamine. Each of them may be used alone or in combination. The base that is inert to the reaction or does not affect the obtaining of the target substance may be used in an amount of typically from about 0.5 to about 10.0 times, and preferably from 0.8 to 3.0 times, the mole of the compound represented by Formula (3).

Purification Step

A reaction product obtained through the reaction step 2 is subsequently subjected to a purification step. The purification step may employ a separation process such as filtration, concentration, distillation, extraction, crystallization, adsorption, recrystallization, or column chromatography, or a separation process as any combination of them.

In a preferred embodiment herein, chlorine-containing compounds are separated and removed using an adsorbent. The adsorbent usable herein is exemplified by silica gel, alumina, activated carbon, magnesia, and hydrotalcite. In particular, silica gel is preferred herein for satisfactory separation and/or removal efficiency. Specifically, the chlorine-containing compounds are most preferably separated and removed using silica gel column chromatography.

In a preferred embodiment of the present invention, the reaction system is washed with water as added, azeotropically dehydrated to a water content of 0.3 percent by weight or less (preferably 0.1 percent by weight or less), and then subjected to crystallization.

Water, if present in crystallization in an amount greater than the range, may cause a fluffy crystal to precipitate

or crystallize more readily as compared with a powdery crystal in a relationship with solubility and may thereby cause the resulting crystal to have a lower bulk density.

In an embodiment, the reaction solvent after the completion of the reaction step 2 is separated and recovered by distillation, and a crystallization solvent is newly added to perform the crystallization. In another embodiment, the reaction solvent is used as the crystallization solvent to perform the crystallization.

The crystallization solvent is exemplified by saturated or unsaturated hydrocarbon solvents such as pentane, hexane, heptane, octane, and petroleum ether; ketones such as methyl ethyl ketone; ester solvents such as methyl acetate, ethyl acetate, isopropyl acetate, and butyl acetate; aromatic hydrocarbon solvents such as benzene, toluene, and xylenes; and ether solvents such as diethyl ether, diisopropyl ether, dibutyl ether, tetrahydrofuran, dioxane, 1,2-dimethoxyethane, and cyclopentyl methyl ether. Alcohols and water cause the decomposition of the unsaturated carboxylic acid amide represented by Formula (1) and are not preferred as the crystallization solvent. In a preferred embodiment of the present invention, the reaction solvent may be used as intact as the crystallization solvent for easy/simple operation and economical efficiency.

The crystallization may be performed by any process such as concentration crystallization, poor-solvent crystallization, and cooling crystallization, alone or in combination.

The crystallization solvent may be used in an amount of typically from about 1 to about 20 times, and preferably from 1 to 15 times, the weight of the unsaturated carboxylic acid represented by Formula (2).

The crystallization may be performed at a temperature of typically from about -5°C to about 70°C, and, in particular, preferably from 10°C to 50°C in concentration crystallization, and preferably from 0°C to 10°C in cooling crystallization.

The crystallization may be performed for a time of typically from about 3 to about 24 hours, whereas the crystallization time may be adjusted as appropriate depending on the reactor scale.

Crystals crystallized by the crystallization operation can be separated and recovered from the crystallization solvent by filtration. The solvent contained in the recovered crystals can be removed by heat drying.

The crystal obtained by the production method contains the unsaturated carboxylic acid amide represented by Formula (1) in a content of 95 percent by area or more, preferably 98 percent by area or more, particularly preferably 99 percent by area or more, and most preferably 99.5 percent by area or more. The crystal, if containing the unsaturated carboxylic acid amide represented by Formula (1) in a content less than the range, may have difficulty in use as a high-performance material. The crystal may have a content of chlorine-containing compounds of preferably about 1000 ppm or less, more preferably 800 ppm or less, and particularly preferably 500 ppm or less, based on the total amount of the crystal. The content of chlorine-containing compounds may be measured typically using combustion ion chromatography. The content of the unsaturated carboxylic acid amide represented by Formula (1) in the crystal may be measured by gas chromatography under analysis conditions as follows.

Gas Chromatography Analysis Conditions

Column: DB-1701
Diameter by length: 0.25 mm by 30 m
Film thickness: 0.25 μm
Split ratio: 1:50
Flow rate: 1 mL/min (He)
Injection volume: 1 μL
Inlet temperature: 250°C
Detector temperature: 280°C
Temperature program: holding at 50°C for 4 min, subsequent temperature rising at a rate of 15°C/min, and subsequent holding at 280°C for 10 min
Analysis time: 30 min

The crystal obtained by the production method may have a bulk density (apparent density) of from 0.2 to 0.7 g/mL, and preferably from 0.3 to 0.7 g/mL.

As used herein the term "bulk density" refers to the ratio of the mass of an untapped (i.e., loose) powder sample to its volume including the contribution of the interparticulate void volume. The bulk density depends on both the density of powder particles and the spatial arrangement of particles in the powder bed. The "bulk density" in the present invention may be determined in the following manner. Into a dry 250-mL graduated cylinder (readable to 2 mL), approximately 100 g ($W_0$ in gram) of a sample weighed with 0.1% accuracy is gently introduced without compacting. The volume ($V_0$ in mL) is read to the nearest graduated unit. The bulk density is calculated by the formula:

$$\text{Bulk density (g/mL)} = W_0/V_0$$

In another preferred embodiment, the crystal according to the present invention may exhibit, in X-ray diffraction, a peak at 2θ in at least one range selected from 6.0 to 8.0, 13.5 to 15.5, and 16.5 to 18.0 and exhibit approximately no peak at 2θ in the ranges of 9.0 to 10.5, 12.5 to 13.5, and 29.0 to 30.0. In particular, the crystal preferably exhibits at least one peak at 2θ in each of the ranges of 6.0 to 8.0, 13.5 to 15.5, and 16.5 to 18.0.

The crystal according to the present invention contains the unsaturated carboxylic acid amide represented by Formula (1) with a high purity, is not bulky, and can be handled excellently (e.g., can be processed and transported excellently). The crystal according to the present invention is advantageously usable as or for high-performance materials such as photosensitive high-performance materials (resist materials) for use in the production of electrical and electronic components. In particular, the crystal is advantageously usable as or for high-performance materials requiring high reliability, such as semiconductor encapsulating materials, packaging materials typically for semiconductor devices and MEMS (micro electro mechanical systems), and photosensitive materials for semiconductor devices, liquid crystal displays, and MEMS.

Examples

The present invention will be illustrated in further detail with reference to an example below. It should be noted, however, that the example is by no means intended to limit the scope of the present invention.

Example 1

In a 50-L reactor, 2.5 kg of 4-methoxycinnamic acid and 11 kg of toluene were placed to give a suspension. This suspension was combined with 1.75 kg of thionyl chloride (1.05 times the mole of 4-methoxycinnamic acid) added dropwise while maintaining the reactor inside temperature at 70±2°C. After the dropwise addition, the reaction was continued until the conversion from 4-methoxycinnamic acid reached 99% or more. After the completion of reaction, about two-tenths of toluene were distilled off together with unreacted thionyl chloride and an acidic gas at 60°C under reduced pressure. After the concentration, the resulting mixture was combined with toluene in an amount equal to the distilled-off amount and yielded 15 kg of a solution of 4-methoxycinnamoyl chloride in toluene.

Next, 1.05 kg of imidazole (1.1 times the mole of 4-methoxycinnamoyl chloride), 1.42 kg of triethylamine (1.0 time the mole of 4-methoxycinnamoyl chloride), 19.5 kg of ethyl acetate were placed and mixed with one another in another reactor. After checking the dissolution of imidazole, the mixture was combined with 15 kg of the 4-methoxycinnamoyl chloride toluene solution added dropwise while maintaining the reactor inside temperature at 30±5°C.

After the completion of dropwise addition, the mixture in the reactor was stirred for one hour and combined with 12.5 kg of ion-exchanged water for washing. The resulting mixture was separated, from which an organic layer was obtained. The obtained organic layer was uninterruptedly washed with 12.5 kg of a 8% sodium hydrogencarbonate aqueous solution, and further washed twice with 12.5 kg of ion-exchanged water. Part of the organic layer after the completion of washing was concentrated, combined with ethyl acetate in an amount of two times the weight of 4-methoxycinnamic acid, and azeotropically dehydrated to a water content of 0.1 percent by weight or less, from which ethyl acetate and toluene were distilled off under reduced pressure. In the midway of the concentration by distilling off, toluene was added in an amount two times the weight of 4-methoxycinnamic acid. Thus, the concentrated substance was obtained as a slurry, cooled down to 5°C or lower, and filtrated to give wet crystals. The wet crystals were dried in vacuo with heating at 60°C and yielded 2.5 kg of crystals including 1-(3-(4-methoxyphenyl)acryloyl)-imidazole with a purity of 100 percent by area.

The obtained crystals were powdery. An aliquot (70 g) of the crystals was weighed and gently introduced into a 250-mL graduated cylinder. The volume was read and found to be 236 mL. Based on this, the bulk density was found to be 0.297 g/mL. Again, 70 g of the crystals were weighed, the volume of which was read by the procedure as above, and was found to be 226 mL. Based on this, the bulk density was found to be 0.310 g/mL. Accordingly, the crystals obtained by the method were found to have an (average) bulk density of 0.30 g/mL.

The obtained crystals were subjected to X-ray diffractometry (XRD). The result thereof is indicated in Fig. 1. The data demonstrate that representative X-ray diffraction peaks were detected at 2θ of 7.3, 14.5, and 17.3; and that no peak was detected at 2θ in the ranges of 9.0 to 10.5, 12.5 to 13.5, and 29.0 to 30.0.

Comparative Example 1

By the procedure of Example 1, 1-(3-(4-methoxyphenyl)acryloyl)-imidazole was produced using 17 kg of 4-methoxycinnamic acid, 76.5 kg of toluene, 11.9 kg of thionyl chloride, 7.1 kg of imidazole, 13.5 kg of triethylamine, and 115 kg of ethyl acetate. However, the organic layer was not subjected to azeotropic dehydration, but, as intact as containing water in a content of 1.5%, subjected to distilling off of ethyl acetate and toluene under reduced pressure, and then to crystallization.

Filtration and drying of crystallized products yielded 17 kg of crystals including 1-(3-(4-methoxyphenyl)acryloyl)-imidazole with a purity of 99.0 percent by area. The obtained crystals were fluffy. An aliquot (20 g) of the crystals was weighed and gently introduced into a 250-mL graduated cylinder, the volume of which was read and found to be 198 mL. Based on this, the bulk density was found to be 0.101 g/mL. Again, 20 g of the crystals were weighed, the volume of which was read by the above procedure, and found to be 197 mL. Based on this, the bulk density was found to be 0.102 g/mL. Accordingly, the crystals obtained by the method were found to have an (average) bulk density of 0.10 g/mL.

The obtained crystals were subjected to X-ray diffractometry (XRD). The result thereof is indicated in Fig. 2. The data demonstrate that representative X-ray diffraction peaks were detected at 2θ of 9.9, 13.0, and 29.5. These peaks were not detected in the crystals obtained in Example 1.

The crystals herein were bulky (had a large volume with respect to a weight) and were inferior in properties in handling, transportation, and charging (filling). For example, the crystals required a larger-sized apparatus in the drying reaction step or, when being dried in plural installments, required a larger number of drying processes; and required a larger number of filling containers in a charging (filling) reaction step, as compared with the crystals obtained in Example 1 upon handling.

The purities of the crystals obtained in the example and the comparative example were determined in the following manner. An aliquot (0.01 to 0.02 g) of crystals to be tested was weighed, diluted with 1.5 to 2 g of acetone (supplied by Wako Pure Chemical Industries, Ltd., JIS Special Grade), and yielded a sample. The sample was subjected to a gas chromatographic measurement to give a chromatogram. The peak area (%) of the target compound was divided by the total sum of peak areas of all components observed in the chromatogram (excluding the peak area detected upon gas chromatographic measurement using acetone alone) to give the purity of the target compound.

The gas chromatographic analysis was performed under conditions as follows.

Analysis Conditions

Column: DB-1701
Diameter by length: 0.25 mm by 30 m
Film thickness: 0.25 μm
Split ratio: 1:50
Flow rate: 1 mL/min (He)
Injection volume: 1 μL
Inlet temperature: 250°C
Detector temperature: 280°C
Temperature program: holding at 50°C for 4 min, subsequent temperature rising at a rate of 15°C/min, and subsequent holding at 280°C for 10 min
Analysis time: 30 min.

The X-ray diffractometry (XRD) of the crystals obtained in the example and the comparative example was performed using an apparatus under conditions as follows.

X-ray diffractometer: MiniFlex II (trade name, supplied by Rigaku Corporation)
X-ray source: Cu Kα radiation, 30 kV, 15 mA
Scan rate: 4.00/min
Divergence slit: 0.625
Scattering slit: 1.25

Industrial Applicability

The crystal according to the present invention has a high purity, is still not bulky, and can be handled excellently (e.g., can be processed and transported excellently). The crystal is therefore particularly useful as raw materials for industrial products. The crystal is advantageously usable as or for pharmaceuticals, agricultural chemicals, polymeric materials, high-performance materials, and intermediates of them. The high-performance materials are exemplified by photosensitive high-performance materials (resist materials) for use in the production of electrical and electronic components. In particular, the crystal is advantageously usable as or for such materials requiring high reliability, such as semiconductor encapsulating materials, packaging materials typically for semiconductor devices and MEMS (micro

electro mechanical systems), and photosensitive materials for semiconductor devices, liquid crystal displays, and MEMS.

**Claims**

1. A crystal comprising
an unsaturated carboxylic acid amide in an amount of 95 percent by area or more,
the crystal having a bulk density of from 0.2 to 0.7 g/mL,
the unsaturated carboxylic acid amide being represented by Formula (1):
[Chem. 1]

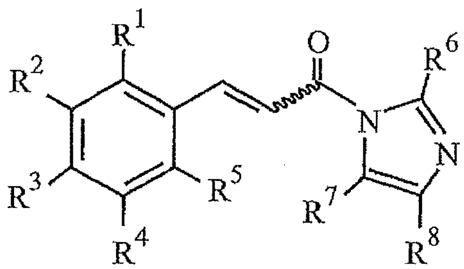

(1)

wherein $R^1$ to $R^5$ are, identically or differently, selected from hydrogen, alkyl, hydroxyl, alkoxy, and nitro, where at least two of $R^1$ to $R^5$ may be linked to form a ring together with carbon atoms constituting the specified aromatic ring; and $R^6$, $R^7$, and $R^8$ are, identically or differently, selected from hydrogen, alkyl, and aryl, where $R^7$ and $R^8$ may be linked to form a ring together with carbon atoms constituting the specified imidazole ring,
the amount of the unsaturated carboxylic acid amide being measured by gas chromatography under conditions as follows:

column: DB-1701,
diameter by length: 0.25 mm by 30 m,
film thickness: 0.25 μm,
split ratio: 1:50,
flow rate: 1 mL/min (He),
injection volume: 1 μL,
inlet temperature: 250°C,
detector temperature: 280°C,
temperature program: holding at 50°C for 4 minutes, subsequently heating at a rate of 15°C/min up to 280°C, and holding at 280°C for 10 minutes, and
analysis time: 30 min.

2. The crystal according to claim 1,
wherein, in X-ray diffraction, the crystal exhibits a peak at 2θ in at least one range selected from the group consisting of 6.0 to 8.0, 13.5 to 15.5, and 16.5 to 18.0, and
wherein, in X-ray diffraction, the crystal exhibits approximately no peak at 2θ in ranges of 9.0 to 10.5, 12.5 to 13.5, and 29.0 to 30.0.

3. The crystal according to one of claims 1 and 2,
wherein the unsaturated carboxylic acid amide comprises at least one compound selected from the group consisting of compounds represented by Formulae (1-1) to (1-6):
[Chem. 2]

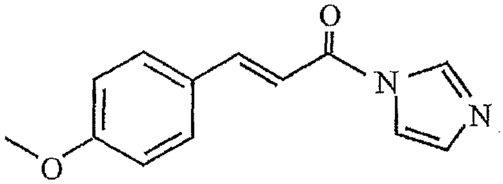

(1-1)

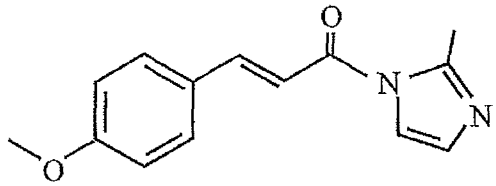

(1-2)

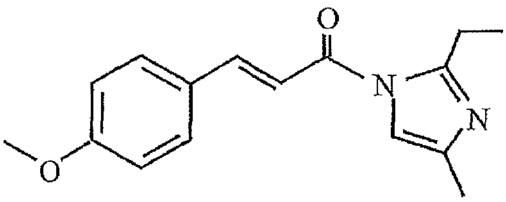

(1-3)

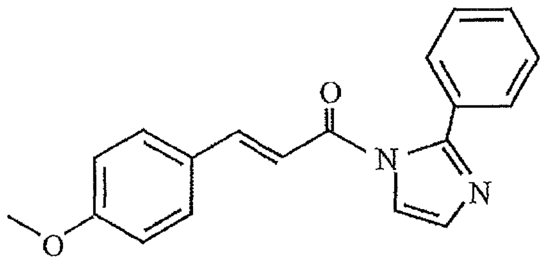

(1-4)

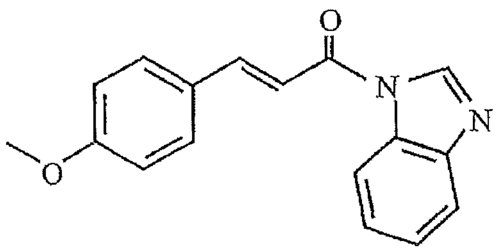

(1-5)

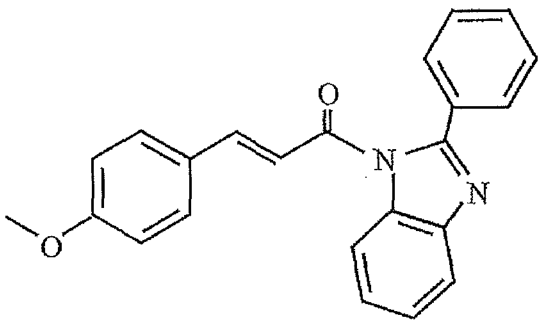

(1-6)

**4.** A method for producing a crystal, the method comprising the steps of:

a) chlorinating an unsaturated carboxylic acid represented by Formula (2) with thionyl chloride to give a compound represented by Formula (3);
b) allowing the prepared compound represented by Formula (3) to react with an imidazole compound represented by Formula (4) to give an unsaturated carboxylic acid amide represented by Formula (1) as a reaction product; and
c) purifying the reaction product to give the crystal according to any one of claims 1 to 3,

the method comprising at least one of:

using the thionyl chloride in an amount of 0.5 to 3.0 times the mole of the unsaturated carboxylic acid represented by Formula (2) in the step a); and
separating and removing a chlorine-containing compound using an adsorbent in the step c),
the step c) comprising the successive substeps of:

adding water to a reaction system to wash the reaction product;
azeotropically dehydrating the reaction product to a water content of 0.3 percent by weight or less; and
subjecting the reaction product to crystallization,

Formulae (2), (3), (4), and (1) expressed as follows:
[Chem. 3]

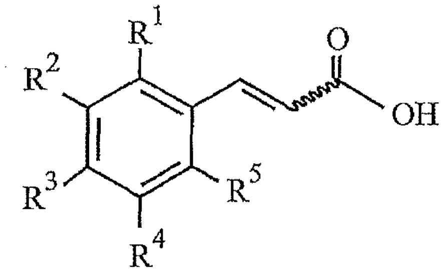

(2)

wherein $R^1$ to $R^5$ are, identically or differently, selected from hydrogen, alkyl, hydroxyl, alkoxy, and nitro, where at least two of $R^1$ to $R^5$ may be linked to form a ring together with carbon atoms constituting the specified aromatic ring,
[Chem. 4]

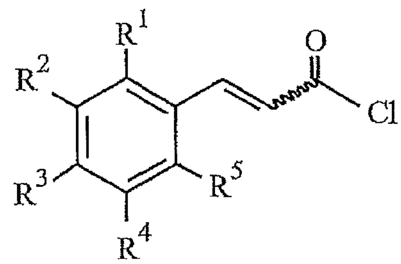

(3)

wherein $R^1$ to $R^5$ are as defined above,
[Chem. 5]

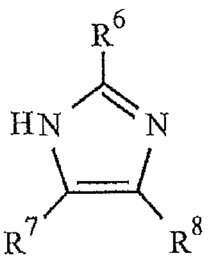

(4)

wherein $R^6$, $R^7$, and $R^8$ are, identically or differently,
selected from hydrogen, alkyl, and aryl, where $R^7$ and $R^8$ may be linked to form a ring together with carbon atoms constituting the specified imidazole ring,
[Chem. 6]

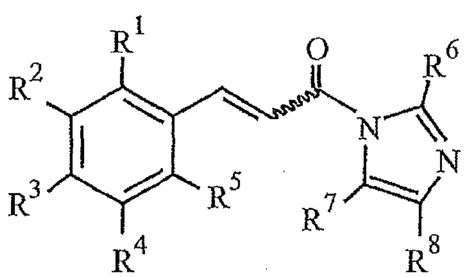

(1)

wherein $R^1$ to $R^8$ are as defined above.

[Fig. 1]

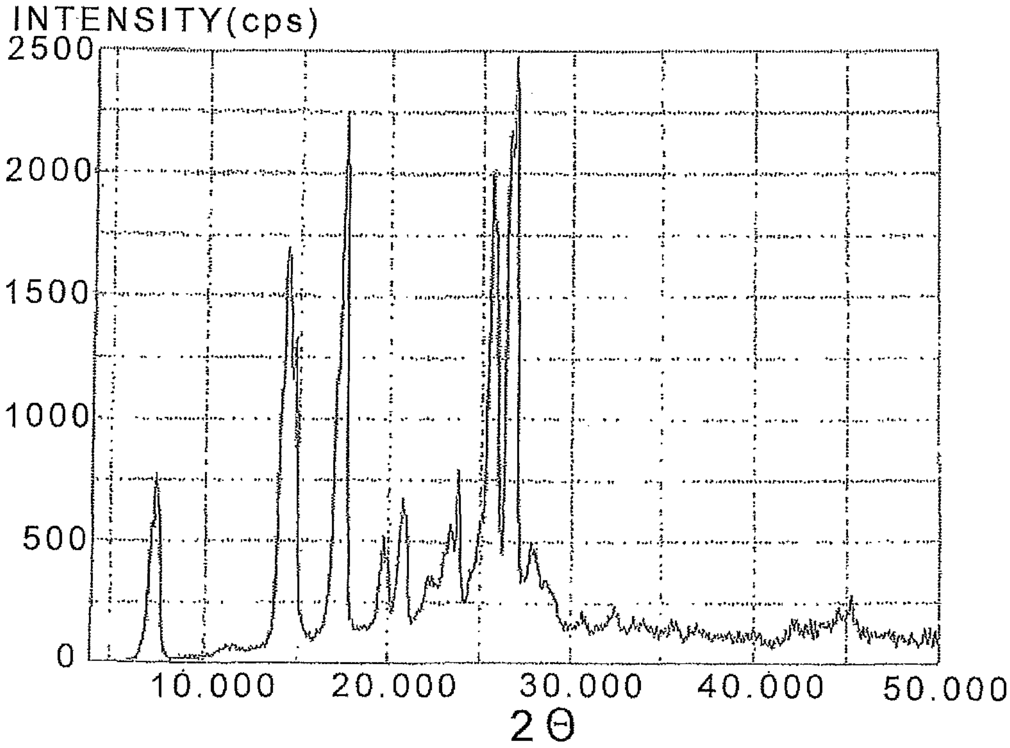
INTENSITY(cps)
2500
2000
1500
1000
500
0
10.000
20.000
30.000
40.000
50.000
2Θ

[Fig. 2]

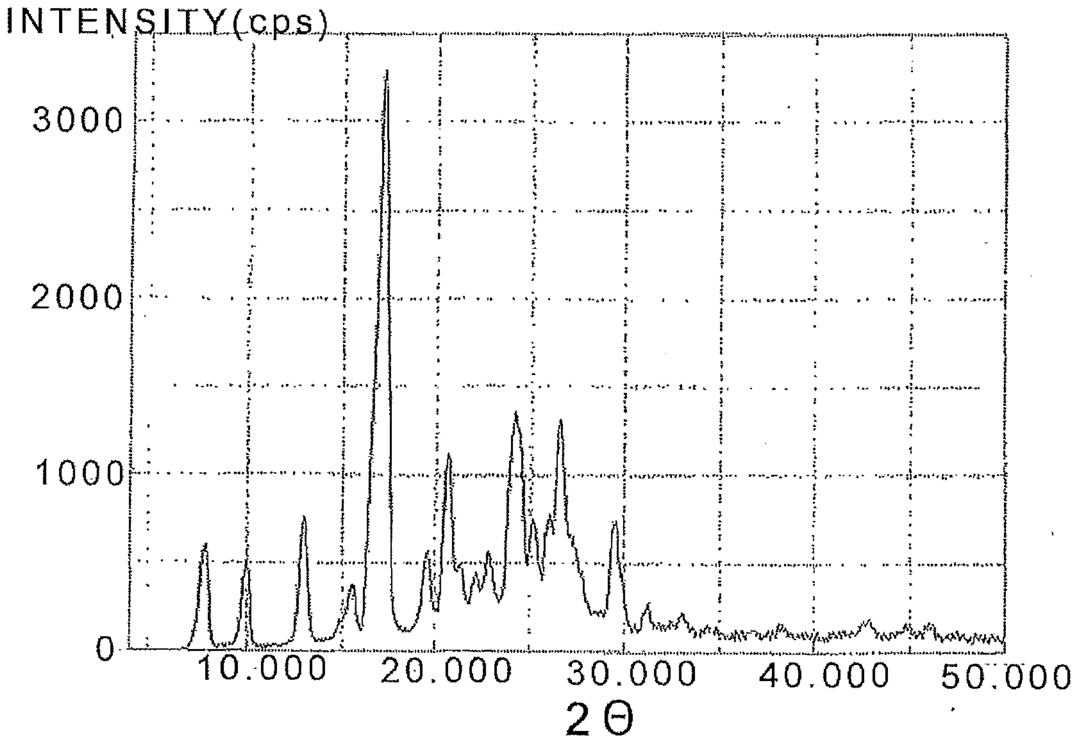
INTENSITY(cps)
3000
2000
1000
0
10.000
20.000
30.000
40.000
50.000
2Θ

**INTERNATIONAL SEARCH REPORT**

International application No.
PCT/JP2013/071147

A. CLASSIFICATION OF SUBJECT MATTER
*C07D233/64*(2006.01)i, *C07D235/18*(2006.01)i

According to International Patent Classification (IPC) or to both national classification and IPC

B. FIELDS SEARCHED

Minimum documentation searched (classification system followed by classification symbols)
C07D233/64, C07D235/18

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>A | Ohta N et al, Journal of Pharmaceutical Sciences, 1983, Vol.72, No.4, p.385-388 | 1-3<br>4 |
| X<br>A | WO 2008/144933 A1 (UNIVERSITE DE MONTREAL), 04 December 2008 (04.12.2008), & US 2010/0204280 A1 | 1-3<br>4 |
| X<br>A | WO 97/29079 A1 (Japan Tobacco Inc.), 14 August 1997 (14.08.1997), & US 6017919 A & EP 887340 A1 & AU 1618697 A & CA 2245586 A | 1-3<br>4 |
| A | JP 62-502749 A (Universite Louis Pasteur), 22 October 1987 (22.10.1987), & US 4931471 A & EP 218622 A & WO 1986/005783 A1 & DE 3680174 C & FR 2579461 A & AT 65078 E | 1-4 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 21 August, 2013 (21.08.13) | 03 September, 2013 (03.09.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

INTERNATIONAL SEARCH REPORT

International application No.
PCT/JP2013/071147

C (Continuation). DOCUMENTS CONSIDERED TO BE RELEVANT

| Category* | Citation of document, with indication, where appropriate, of the relevant passages | Relevant to claim No. |
|---|---|---|
| A | JP 2005-517737 A (Council of Scientific and Industrial Research), 16 June 2005 (16.06.2005), & US 2003/0161860 A1 & EP 1478632 A1 & WO 2003/070713 A1 & AU 2002234820 A | 1-4 |
| A | WO 2009/123122 A1 (Dainippon Printing Co., Ltd.), 08 October 2009 (08.10.2009), & JP 2011-89102 A & US 2011/0086311 A1 & CN 101981154 A & KR 10-2011-0002018 A | 1-4 |
| P,A | WO 2012/176621 A1 (Daicel Corp.), 27 December 2012 (27.12.2012), & JP 2013-6795 A & TW 201302672 A | 1-4 |
| P,A | WO 2012/176693 A1 (Daicel Corp.), 27 December 2012 (27.12.2012), & TW 201305732 A | 1-4 |

Form PCT/ISA/210 (continuation of second sheet) (July 2009)

INTERNATIONAL SEARCH REPORT

International application No.
PCT/JP2013/071147

With respect to the specification of the compound crystal of the present invention with use of the value of the X-ray diffraction peak in claim 2, formula (1) in claim 1 includes a large number of compounds in addition to the specific compounds in the examples of the present invention since compounds having different chemical structures naturally have different X-ray diffraction peak values.

Since there is no explanation of the relation between the X-ray diffraction peak value set forth in claim 2 and a chemical structure or a crystal form in the description, it cannot be understood what kind of compounds or what kind of crystal forms among the compounds set forth in claim 1 are intended to be specified by the above-described definition of the X-ray diffraction peak value.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009123122 A
- JP 2002187868 A
- JP 2009263543 A


### Non-patent literature cited in the description


- *Journal of Organic Chemistry,* 2004, vol. 69, 4216
- *ournal of American Chemical Society,* 1950, vol. 72, 3885
- *Synthesis,* 1989, 598